# EUROPEAN PATENT APPLICATION

(11) **EP 4 219 711 A1**
(43) Date of publication of application: **02.08.2023**
(21) Application number: 22154018.0
(22) Date of filing: 28.01.2022
(51) Int. Cl.: C12N 15/113, A61K 31/712

(54) **ANTISENSE COMPOUNDS FOR THE TREATMENT OF CORONAVIRUS INFECTION**

(71) Applicant: Ceinge Biotecnologie Avanzate SCarl, 80145 Napoli (IT); Zollo, Massimo, 80131 Napoli (IT); Ferrucci, Veronica, 80055 Portici (NA) (IT); de Antonellis, Pasqualino, 83100 Avellino (IT)
(72) Inventor: ZOLLO, Massimo, 80131 NAPOLI (IT); FERRUCCI, Veronica, 80055 PORTICI (NA) (IT); DE ANTONELLIS, Pasqualino, 83100 AVELLINO (IT)
(74) Representative: Bianchetti & Minoja with Trevisan & Cuonzo IPS SRL

(57) **Abstract**

There are disclosed antisense oligonucleotides targeting a region of subgenomic RNA encoding nucleoprotein N (sgN) of coronavirus, compositions thereof and their uses for prevention and treatment of coronavirus infection.

## Description

The present invention relates to antisense oligonucleotides for treating coronavirus infections, compositions and uses thereof.

### Background of the invention

Human CoVs are members of the Nidovirales order and belong to the Coronaviridae family. To date, seven species of human CoVs have been described: HCoV-NL63, HCoV-229E, HCoV-OC43, HCoVHKU1, SARS-CoV, MERS-CoV, and SARS-CoV-2. Like other CoVs, SARS-CoV-2 is an enveloped virus with a positive-sense, single-stranded RNA genome. SARS-CoV-2 belongs to the genus betacoronavirus, together with SARS-CoV and MERS-CoV (with 80% and 50% percentage identity, respectively).

Coronaviruses, including SARS-CoV-2, have the largest genomes (26-32 kb) among all of the RNA virus families, which are flanked by 5' and 3' untranslated regions. SARS-CoV-2 RNA contains a common 'leader' sequence (of 70 nt). Upon cell entry, the viral genomic RNA (gRNA) is translated to produce nonstructural proteins from two large open reading frames (ORFs), ORFla and ORF1b, via proteolytic cleavage. Among these, 15 nonstructural proteins make up the viral replication and transcription complex. Of importance, Nsp12 (which harbors RNA-dependent RNA polymerase; RdRp) leads the viral replication and transcription mechanisms by using viral RNA as the template.

A hallmark of CoVs is a "discontinuous transcription" mechanism that produces a set of subgenomic RNAs (sgRNAs). Indeed, during their viral cycle, CoVs replicate their genomic RNA to produce full-length negative-sense RNA molecules that act as the templates for the synthesis of positive-sense gRNAs that are then packaged by the structural proteins into newly assembled virions. However, the ORFs are transcribed from the 3' one-third of the genome to form sgRNAs that encode the SARS-CoV-2 structural proteins (i.e., spike [S], envelope [E], membrane [M], nucleocapsid [N]), and several accessory proteins (e.g., 3a, 6, 7a, 7b, 8, 10), according to the "leader-to-body fusion" model. Briefly, during the negative-strand synthesis, the viral replication and transcription complex interrupts transcription when it crosses a transcription regulatory sequence (TRS) upstream of most ORFs in the 3' one-third of the viral genome (i.e., a TRS 'body', or TRS-B). The synthesis of the negative-strand RNA is then re-initiated at the TRS in the leader sequence (TRS-L) at 70 nucleotides from the 5' end of the genome, due to the interaction between TRS-B of the negative-sense nascent RNA and TRS-L of the positive-sense gRNA. Upon re-initiation of RNA synthesis at the TRS-L region, a negative-strand copy of the leader sequence is added to the nascent RNA to complete the synthesis of negative-strand sgRNAs. These fused negative-strand intermediates are used as templates to synthesize positive-sense sgRNAs that are translated into both structural and accessory proteins.

To date, eight main sgRNAs have been reported to be produced in SARS-CoV-2-infected cells. In addition to these canonical sgRNAs, noncanonical RNA products of discontinuous transcription have also been reported for SARS-CoV-2, including fusions of the 5' leader sequence to unexpected 3' sites, TRS-L-independent long-distance fusions, and local fusions that result in small deletions mainly in structural and accessory genes.

Of interest, the N protein sgRNA (sgN; which codes for the N protein) is the most abundant sgRNA during viral infection, mostly due to the low DG value in the duplex formation between TRS-L and TRS-B. On this basis, of all of the sgRNAs, sgN has been shown to be the most abundant in SARS-CoV-2-infected cells. Furthermore, together with sgRNA for Orf7a, the same sgN also shows highest abundance in swab samples from COVID19-affected patients. Another important finding related to the SARS-CoV-2 N protein encoded by the sgN transcript is its role in the regulation of the discontinuous transcription process through its C-terminal domain, which retains its interaction with TRS sequences, and the consequential regulation of transcription. To this end, early and continuous expression of the sgN transcript ensures the generation of the N protein in multiple copies in nascent viral genome particles, which is of great importance for SARS-CoV-2 replication and propagation.

To date, a siRNA-based approach that targets the leader sequence of SARS-CoV (Li et al, 2005 doi: 10.1038/sj.gt.3302479) and a chemical inhibitor that targets the RNA-binding affinity of N protein (Lin SY et al., 2014; doi: 10.1021/jm500089r) have been tested *in vitro* only against SARS-CoV infection. The effectiveness of these therapeutic approaches against SARS-CoV-2 have not been tested to date. Further, there are no prophylactic treatments available.

### Description of invention

It was surprisingly found that targeting the sgN sequence with antisense oligonucleotides can impair viral replication and syncytia formation in human cells. In particular, oligonucleotides designed against the junction site between the leader sequence, the transcription regulating sequences (TRSs) and the 5' end of gene N proved effective in reducing virus load and propagation in SARS-CoV-2 infected cells.

Accordingly, the invention is directed to an antisense oligonucleotide having a sequence complementary to a region of SARS-CoV2 subgenomic RNA encoding nucleoprotein N (sgN), said region consisting of a leader sequence (LS), two transcription-regulatory sequences (TRS), namely TRS(leader) and TRS(body), and a 5' portion of gene N ("N"), such as:

5'-LS-TRS(leader)-TRS(body)-N-3'

wherein:
- LS is UCGAUCUCUUGUAGAUCUGUUCU (SEQ ID NO:1) or a fragment thereof including UCU at the 3'end
- TRS(leader) is CUAAACGAAC (SEQ ID NO:2)
- TRS(body) is selected from AAACUAAA; CAAACUAA and AAACUUAA
- N is selected from AUGUCUGAUAAUGGAC (SEQ ID NO:3) and AUGUCUCUAAAUGGAC (SEQ ID NO:4).

In a preferred embodiment, the oligonucleotide is complementary to a region 5'-LS-TRS(leader)-TRS(body)-N as defined above, wherein:
- LS is UCU
- TRS(leader) is CUAAACGAAC (SEQ ID NO:5)
- TRS(body) is AAACUAAA
- N is AUGUCUGAUAAUGGAC (SEQ ID NO:6).

As herein used, the term "oligonucleotide" denotes oligomers of deoxyribonucleic or ribonucleic acid in which the bases are selected from G, C, A, U (RNA) and T (DNA), or derivatives thereof as disclosed in the following.

The antisense oligonucleotide sequence may consist of from 20 to 40, preferably from 30 to 40 bases.

In a preferred embodiment, the antisense oligonucleotide consists of the sequence GXCCAXXAXCAGACAXXXXAGXXXGXXCGXXXAGAGA (SEQ ID NO:7)
wherein "X" is, independently from one another, U or T. More preferably it consists of a sequence which is selected from
GUCCAUUAUCAGACAUUUUAGUUUGUUCGUUUAGAGA (SEQ ID NO:8)
   and
GTCCATTATCAGACATTTTAGTTTGTTCGTTTAGAGA (SEQ ID NO:9).

The oligonucleotide backbone may be modified such as to comprise bonds and/or structural moieties that prevent degradation or cleavage mechanisms that may occur in the cells - e.g. mediated by nucleases - or during storage of the product in delivery vehicles or media. The backbone modifications may also contribute to increase stability, reduce off-target effects and cytotoxicity, and improve uptake by the cell. In particular embodiments, the oligo(ribo)nucleotide backbone includes a modified nucleotide which is selected from phosphorodiamidate morpholino (PMO) nucleotide, 5' -phosphorothioate nucleotide, 5-methylated cytosine nucleotide, 2'-O-methyl nucleotide, 2'-O-methoxyethyl nucleotide, 2'-fluoro nucleotide, 3'-nitrogen nucleotide; or said backbone includes a locked nucleic acid (LNA) or a peptide nucleic acid (PNA).

A backbone carrying 2'-O-methyl-(ribo)nucleotides is preferred.

Another aspect of the invention relates to a pharmaceutical composition comprising an antisense oligonucleotide as herein disclosed. The composition is preferably designed for delivery of the oligonucleotide molecule to the respiratory tract and to the lungs and it is administered via nasal or pulmonary routes. Suitable forms include aerosol, spray, solutions or suspensions to be administered with a nebulizer.

In alternative, it can be orally or parenterally administered, e.g. through intravenous, intramuscular, subcutaneous routes.

In manufacturing the pharmaceutical composition, the oligonucleotide, preferably one carrying a modified backbone, can be dissolved or dispersed in an essentially aqueous solution, which can be added with cosolvents, or it can be formulated with suitable vehicles, carriers and excipients.

The oligoribonucleotide can be encapsulated in lipidic or polymeric (nano)particles or liposomes or micelles prior to its addition to the vehicle or mixture with carriers and excipients. Preferably it is loaded into stable nucleic acid lipid particles (SNALP) using the methods and technology described in De Antonellis et al., 2013 (DOI 10.1007/s00210-013-0837-4). Other suitable non viral vectors include lipoplexes (cationic liposomes), solid lipid nanoparticles and exosomes (Zoulikha Makhloufi et al., https://doi.org/10.1016/j.apsb.2021.08.009).

In an alternative embodiment, the oligo(ribo)nucleotide is delivered to the target cells by means of viral vectors. To this end, the oligomeric nucleic acid sequence is inserted in a suitable genetic construct or expression cassette, including genetic elements such as promoters, ITR sequences and other elements that may be required by the type of viral vector used. The latter can be a retrovirus, e.g. a lentivirus, an adenovirus or an adeno-associated virus (AAV).

In a further embodiment, the invention provides an oligonucleotide or a composition thereof as herein disclosed, for use in the prevention or treatment of a coronavirus infection.

According to the invention, "coronavirus" is an RNA virus with a gene size of 27 to 32 kb which belongs to the family Coronaviridae and subfamily Coronavirinae that consists of four genera (alpha, beta, gamma, and delta). There are currently 7 types of coronaviruses as shown in Table 1 below, which are infectious to humans. These types are divided into the types 229E, OC43, NL63, HKU1 that cause cold and the types SARS-CoV, SARS-CoV2, MERS-CoV that cause severe pneumonia.

**Table 1**

| Virus name | Genus | Host | Symptom |
|---|---|---|---|
| HCoV-229E | Alpha | Human | Mild respiratory symptoms |
| HCoV-NL63 | Alpha | Human | Mild respiratory symptoms |
| SARS-CoV | Beta | Human | Severe respiratory symptoms |
| MERS-Co V | Beta | Human | Severe respiratory symptoms |
| HCoV-OC43 | Beta | Human | Mild respiratory symptoms |
| HCoV-HKU1 | Beta | Human | Pneumonia symptoms |
| SARS-CoV-2 | Beta | Human | Mild respiratory symptoms, causing shortness of breath in severe cases |

The disease caused by the coronavirus infection may be coronavirus enteritis, coronavirus diarrhea, severe acute respiratory syndrome (SARS), Middle East respiratory syndrome (MERS), or a combination thereof. Preferably, the infection disease is the "severe acute respiratory syndrome virus-2 (SARS-CoV-2)", wherein the causative agent is SARS-CoV-2. However, any disease may be included without limitation as long as such a disease can be caused by the coronavirus infection.

The disclosed antisense compounds or compositions can be administered in an effective dosage for the prophylactic or therapeutic treatment of coronavirus infection. Dosage amounts will depend on factors like sex, age and general health conditions of the subject, comorbidity states, severity of infection or amount of risk of developing an infection, and they will typically be in the range from 0.125 mg/Kg to 5 mg/Kg. The total daily dosage may typically range from 0.250 mg/Kg to 10mg/Kg.

### Description of the Figures

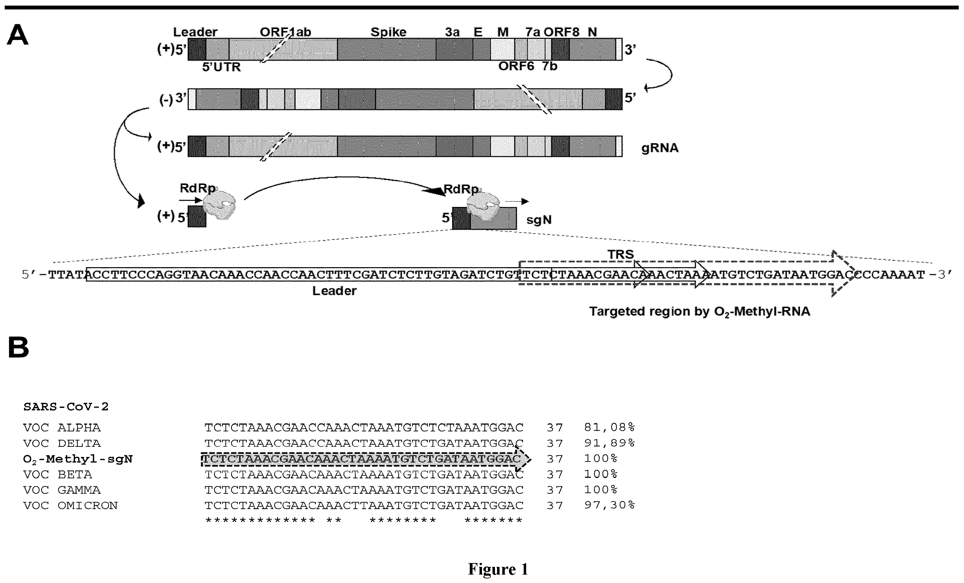

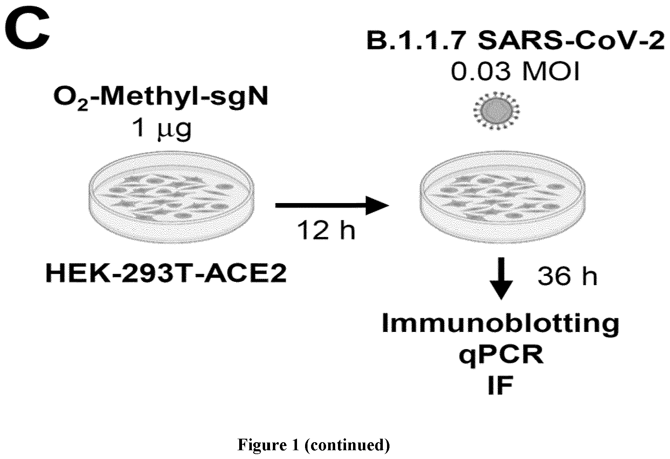

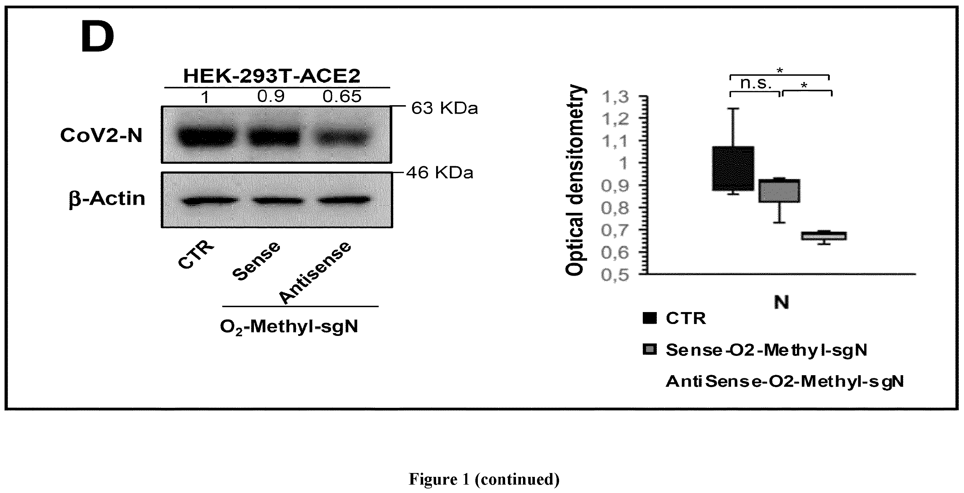

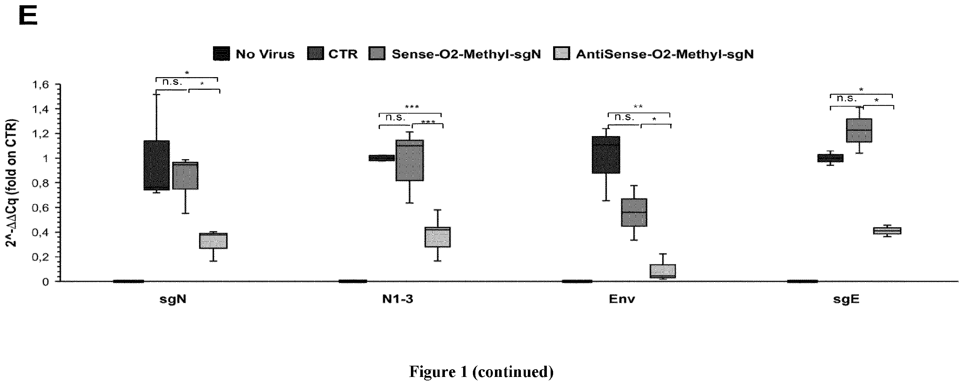

**Figure 1****. Reduction of viral load by targeting sgN in HEK293T-ACE2 SARS-CoV-2-infected cells. (A)** Schematic representation of the SARS-CoV-2 genome organization, the canonical sgRNAs biogenesis (according to the "leader-to-body fusion" model) and the 2'-O-methyl antisense RNA design. During its viral cycle, SARS-CoV-2 virus replicates its positive sense (+) genomic RNA (29,903 nt) to produce full-length negative-sense (-) RNA molecules that act as templates for the synthesis of new positive-sense (+) gRNAs, that are then packaged into newly assembled virions. However, according to the "leader-to-body fusion" model (Kim D et al., 2020; doi: 10.1016/j.cell.2020.04.011), during negative-strand synthesis, the replication complex (including RdRp) interrupts the transcription when it crosses a transcription-regulatory sequence (TRS) located upstream to most ORFs (including gene N) in the 3' one-third of the viral genome (TRS 'body', or TRS-B). Thus, the synthesis of the negative-strand (-) RNA is re-initiated at the TRS in the leader sequence (TRS-L, red box) at 70 nucleotides from the 5' end of the genome. Through this mechanism, a negative strand copy of the leader sequence is added to the nascent RNA to complete the synthesis of negative-strand (-) sgRNAs. These negative RNAs are then used as templates to synthesize positive-sense (+) sgRNAs that are translated into both structural and accessory proteins. 2'-O-Methyl antisense RNA (37 bp, yellow arrow with dashed lines) was developed based on the sequence of sgN (i.e., leader sequence [in red], TRS upstream to gene N [in green], and 5' end of gene N). **(B)** Sequence alignment of the regions recognized by 2'-O-methyl RNA sgN among SARS-CoV-2 VOC Alpha, Beta, Gamma, Delta, and Omicron, illustrating their degrees of percentage identity (i.e., VOC Alpha: 81.1%; Beta: 100%; Gamma: 100%; Delta: 91.9%; Omicron: 97.3%). These sequence alignments were realized using the Clustal Omega software. **(C)** Experimental plan. Human HEK-293T cells overexpressing ACE2 were plated and transfected with 2'-O-methyl antisense or sense RNA against sgN. Nontransfected cells were used as the negative control. After 12 h, the cells were infected with 20I/501Y.V1 (B.1.1.7) (UK) (EPI_ISL_736997) SARS-CoV-2 viral particles (MOI, 0.03), and noninfected cells were used as the negative control for infection. At 36 h from infection (i.e., 48 h from transfection), the cells were lysed or fixed (in 4% paraformaldehyde) for protein/RNA extraction or immunofluorescence, respectively. **(D)** Left: Representative immunoblot (using antibodies against the indicated proteins) of human HEK-293T cells overexpressing ACE2 transfected with 2'-O-methyl antisense or sense RNA against sgN and then infected with 20I/501Y.V1 (B.1.1.7) (UK) (EPI_ISL_736997) SARS-CoV-2 viral particles (MOI, 0.03) for a further 36 h. β-Actin was used as the loading control. All experiments were performed in triplicate. Right: Densitometry analysis of the indicated band intensities in blots from three independent experiments. Data are means ±SD. ^{∗}P <0.05 (unpaired two-tailed Student's t test; n = 3 independent experiments per group). Negative controls: nontransfected cells (CTR). N.S., not significant. **(E)** Quantification of mRNA abundance relative to that in control (CTR) cells (2-ΔΔCt) for sgN, sgE, N1-3 and gene E. RT-PCR analysis of RNA extracted from cells treated as described in **(C)**. Noninfected cells and SARS-CoV-2-infected cells not transfected (CTR) were used as controls. Data are means ±SD. ^{∗}P <0.05, ^{∗∗}P <0.01, and ^{∗∗∗}P <0.001 (unpaired two-tailed Student's t test; n = 3 independent experiments per group).

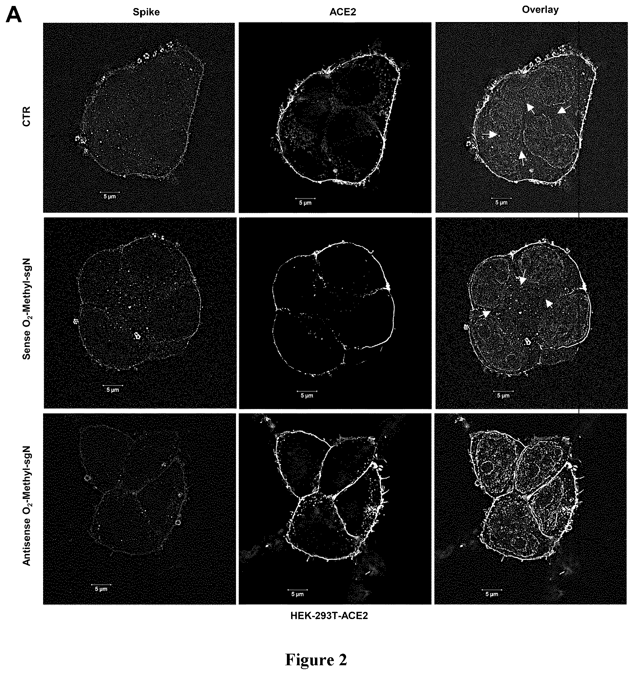

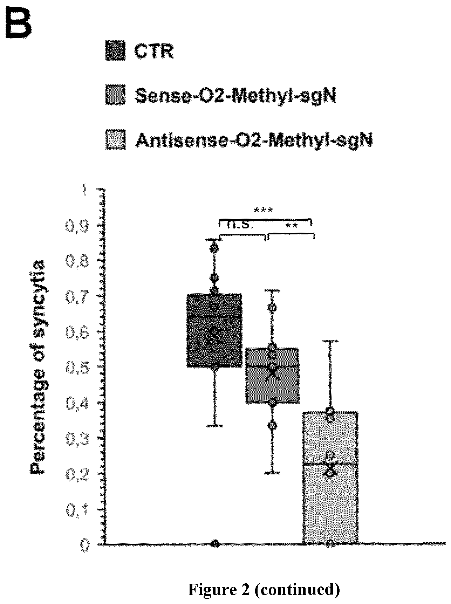

**Figure 2****. Reduction of syncytia formation by targeting sgN in HEK293T-ACE2 SARS-CoV-2-infected cells. (A)** Immunofluorescence staining with an antibody against the viral S protein (green) and human ACE2 protein (red) in human HEK-293T cells overexpressing ACE2 transfected with 2'-O-methyl antisense or sense RNA against sgN for 12 h and then infected with 20I/501Y.V1 (B.1.1.7) (UK) (EPI_ISL_736997) SARS-CoV-2 viral particles (MOI, 0.03) for a further 36 h. SARS-CoV-2-infected cells not transfected (CTR) were used as the negative control. Representative images of syncytia. DAPI was used for nuclei. The SIM images were acquired with Elyra 7 and processed with the Zeiss ZEN software (blue edition). Magnification, ×63. Scale bars: 5 µm. **(B)** Comparison of the proportions (%) of syncytia counted by SIM fluorescence microscopy in human ACE2 (red) proteins in human HEK-293T cells overexpressing ACE2 transfected with 2'-O-methyl antisense or sense RNA against sgN for 12 h and then infected with 20I/501Y.V1 (B.1.1.7) (UK) (EPI_ISL_736997) SARS-CoV-2 viral particles (MOI, 0.03) for a further 36 h. SARS-CoV-2-infected cells not transfected (CTR) were used as the negative control. Data are means ±SD. ^{∗∗}P <0.01, and ^{∗∗∗}P <0.001 (unpaired two-tailed Student's t test; n = 3 independent experiments per group). More than 60 nuclei were counted per condition.

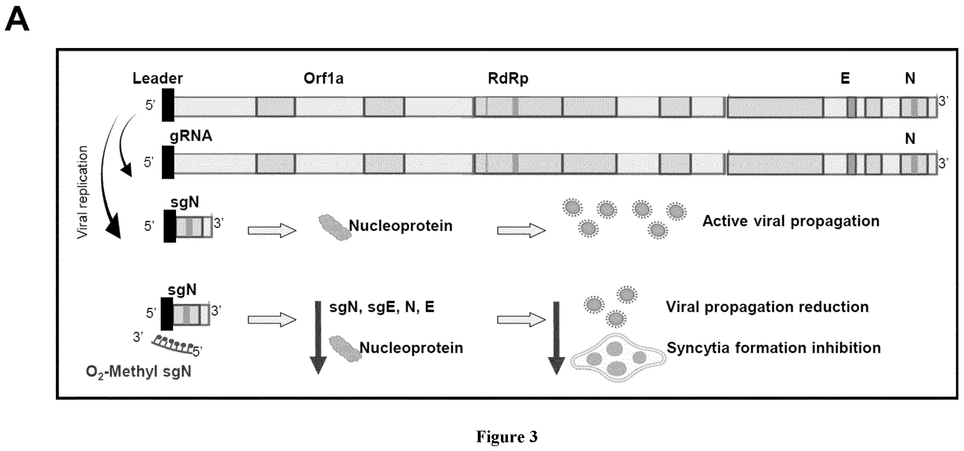

**Figure 3****. Proposed mechanism of action of sgN during infection by SARS-CoV-2. (A)** The biogenesis of subgenomic RNAs follows the "leader to TRS-B fusion model", which produces copies of sgN that are translated to the N protein. Impairment of this process through 2'-O-methyl antisense RNA against sgN results in therapeutic benefits in terms of decreased viral replication and syncytia formation *in vitro.*
**Figure 4**
   HEK-293T overexpressing ACE2 cellular model.
   (A) Quantification of mRNA abundance relative to that of HEK-293T cells (2-ΔΔCt) for the ACE2 gene. RT-PCR analysis with SYBR Green of RNA extracted from HEK-293T wildtype (WT) and HEK-293T-overexpressing ACE2 cells. Data are means ±SD. ^{∗∗∗}P <0.001 (unpaired two-tailed Student's t test; n = 3 independent experiments per group). (B) Representative immunoblotting (using antibodies against the indicated proteins) of human HEK-293T cells and HEK-293T stable clones overexpressing ACE2. B-Actin was used as the loading control. (C) Immunofluorescence staining with an antibody against the human ACE2 (plasma membrane localization signal) protein in human HEK-293T cells and HEK-293T stable clones overexpressing ACE2. DAPI was used for nuclei. The SIM image was acquired with Elyra 7 and processed with the Zeiss ZEN software (blue edition). Magnification, ×63. Scale bars: 5 µm.
**Figure 5**
   2'-O-methyl RNA against sgN sequence. Related to Figure 3A-B. (A) Sanger sequencing of the sgN region of SARS- CoV- 2. Black arrow indicates the leader sequence, dark gray arrow indicates the TRS upstream gene N, and light gray arrow indicates gene N. The region recognized by 2'-O-methyl antisense RNA is highlighted in the light gray box. (B, C) Clade 21J is now the predominant form of Delta, with an estimated ~90% global frequency. (D) Frequencies of variant nucleotides calculated on a collection of about 350,000 sequences. Dataset downloaded from Global Initiative on Sharing Avian Influenza Data (GISAID) on November 15, 2021.

### Examples

### Example 1 - 2'-OMe-oligonucleotide RNA oligo synthesis and purification

The 2'-OMe-oligonucleotides were synthesized on a Millipore Cyclone Plus DNA synthesizer at a 1 µmol scale using commercially available 5'-O-(4,4'-dimethoxy- trityl)-2'-OMe-3'-O-(2-cyanoethyl-N,N-diisopropyl) RNA phosphoramidite monomers and 2'-OMe RNA SynBase CPG 1000/110 as the solid-phase support (Link Technologies). The sequence of the 02-Methyl RNA oligo (2'-OMe is inserted into each following nucleotide) against sgN is: GUCCAUUAUCAGACAUUUUAGUUUGUUCGUUUAGAGA (SEQ ID NO:8). The oligomers were detached from the support and deprotected by treatment with concentrated aqueous ammonia for 12 h at 55 °C. The combined filtrates and washings were concentrated under reduced pressure, dissolved in H₂O, and analyzed and purified by anion exchange high-performance liquid chromatography on a NUCLEOGEL SAX column (Macherey-Nagel, 1000-8/ 46) using buffer A: 20 mM KH₂PO₄/K₂HPO₄ aqueous solution (pH 7.0), containing 20 % (v/v) CH₃CN; and buffer B: 1 M KCl, 20 mM KH₂PO₄/K₂HPO₄ aqueous solution (pH 7.0), containing 20 % (v/v) CH₃CN. These were used as a linear gradient from 0% to100% Bover 45min at a flow rate of 1 ml/min. The purified oligomers were successively desalted using Sep-Pak C18 cartridges (Waters) and characterized by electroionization mass spectrometry.

### Example 2 - Preparation and characterization of stable nucleic acid lipid particles (SNALPs) containing O2-Methyl RNA oligo against sgN

Lipid stock solutions were prepared in ethanol. Typically, 6.5 µmol total lipid (0.2 ml total volume) was added to a glass tube. In a separated tube, 1 mg O2-Methyl RNA oligo against sgN was dissolved in 0.3 ml 20 mM citric acid, pH 4.0. The two solutions were warmed for 2-3 min at 65 °C, and the lipid solution was quickly added to the O2-Methyl RNA oligo solution while stirring. The mixture was passed 30 times through three-stacked 100-nm polycarbonate filters using a thermobarrel extruder (Northern Lipids Inc., Vancouver, BC, Canada) maintained at approximately 65 °C. Nonencapsulated 02-Methyl RNA oligo was removed by chromatography using a Sephadex G-50 DNA grade column (GE Healthcare, Milan, Italy). A typical 0.5 ml formulation contained 6.5 µmol DSPC/cholesterol/DODAP/PEG-Cer16 (25 mol/45 mol/20 mol/10 mol) and 1 mg O2-Methyl RNA oligo. 1,2-Dioleyl-3-dimethylammonium propane (DODAP) and N-palmitoyl-sphingosine-1-succinyl [methoxy(polyethylene glycol)2000] (PEG-Cer16) were purchased from Avanti Polar Lipids. Disteroylphosphatidylcholine (DSPC) was kindly donated by Lipoid GmbH (Cam, Switzerland). Cholesterol, sodium chloride, potassium chloride, sodium phosphate, HEPES, citric acid, and sodium citrate were purchased from Sigma-Aldrich (Milan, Italy). Ethanol and other reagents were obtained from Carlo Erba Reagenti (Milan, Italy). The encapsulation efficiency was about 80 %. The mean diameter and size distribution of the SNALPs were determined at 37 °C by Nano-Zs (Malvern, UK). Each sample was diluted in phosphate-buffered saline (PBS), filtered with 0.22-µm pore size polycarbonate filters (MF-Millipore, Microglass Heim, Naples, Italy), and analyzed with the detector angle at 173°. The polydispersity index was measured to determine the particle size distribution. For each batch, the diameter and size distributions were calculated as the means of three measures. For each formulation, the diameter and polydispersity index were calculated as the means of two different batches. The 1 µg of 02-Methyl RNA oligo against sgN encapsulated in the SNALPs were measured spectrophotometrically. Briefly, 2 µl SNALP suspension was analyzed at 260 nm using a nanoprobe (Thema Research, Bologna, Italy), and the encapsulation efficiencies were calculated as the ratio of the initial and final oligodeoxynucleotide-to-lipid ratios (×100).

### Example 3 - Delivery into the cells of SNALPs containing O2-Methyl RNA oligo against sgN through a nonambulatory nebulizer system

1 mg 02-Methyl RNA oligo against sgN (Sequence: GUCCAUUAUCAGACAUUUUAGUUUGUUCGUUUAGAGA (SEQ ID NO:8)) encapsulated into SNALPs was dissolved in 2 ml of water or 0,9 % NaCl solution and nebulized for 2 min with a nebulizer system (mesh nebulizer; YM-252; input, 5 V/1 A; oscillation frequency, 110 kHz; atomized particles, <5 mM; condensation rate, 1 ml/min). After removal of the medium from the flask, the nebulized SNALPs-O2-Methyl RNA oligo against sgN was directed into the flask. The medium was then replaced after the nebulizing. Those cells nebulized with SNALPs-O2-Methyl RNA oligo against sgN were then infected with viral particles belonging to 20I/501Y.V1 (B.1.1.7) clades, for 48 hours.

### Example 4 - Reduction of viral load by targeting sgN in HEK293T-ACE2 SARS-CoV-2-infected cells

SARS-CoV-2 has been now reported to induce systemic perturbations by impacting multiple organs. In this regard, lung is the primary target for SARS-CoV-2 that causes an early respiratory infection. Then, mostly due to the wide expression of ACE2 in a heterogeneous population of systemic cells, SARS-CoV-2 can damage several systemic tissues and result in multi-organ dysfunction, including kidney due to high ACE2 expression levels. HEK-293T cells have been widely used as a cellular model to identify seral mechanisms of action. Here, in order to allow and normalize SARS-CoV-2 infection, we have generated HEK-293T stable cell clones overexpressing human ACE2 cDNA under the control of CMV promoter. This cellular model overcomes those alterations in the viral infection efficiency due to the different multiplicity of infection (MOI). Furthermore, at this time, kidney cells (including HEK-293 expressing ACE2) are used for in vitro model platforms for SARS-CoV-2 infection.

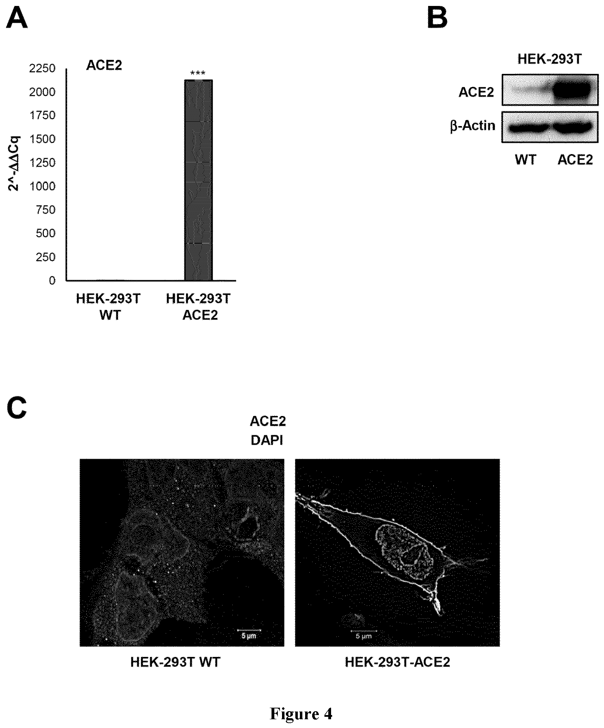

In this regard, we first generated the HEK293T-ACE2 cellular model by creating stable clones using a PCDNA3.1 +C-DYK (Addgene vector name: pCEP4-myc-ACE2: containing ACE2 cDNA human wild-type). After antibiotic selection for a hygromycin resistance gene, several clones were selected. One clone here (Fig. 4A) contained a high copy number endogenous plasmid that expressed the ACE2 protein (2,000-fold compared to HEK293T wild-type cells), with expression of a significant amount of the protein in the membranes of these cells (Fig. 4A-C).

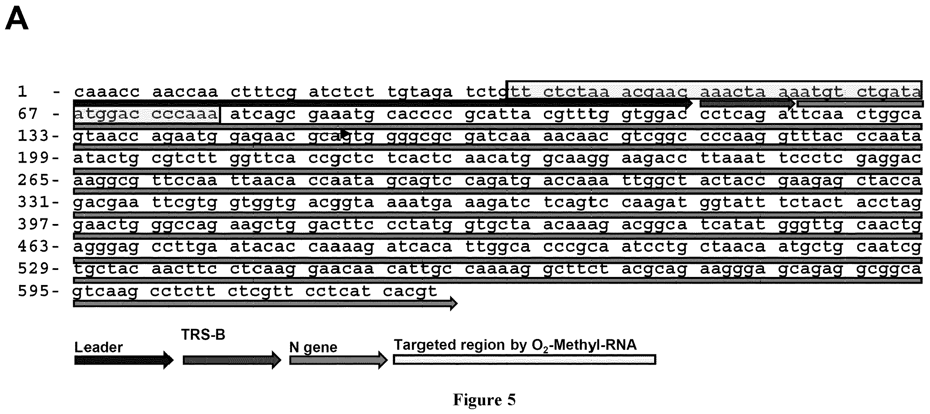

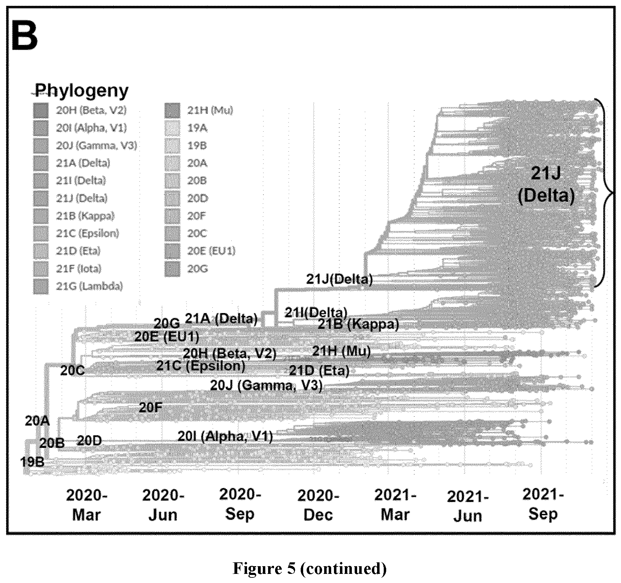

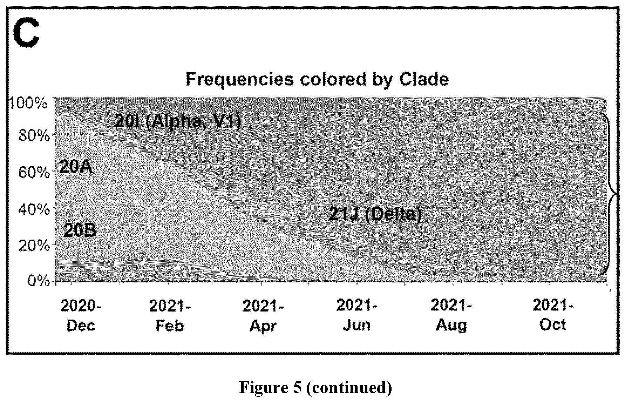

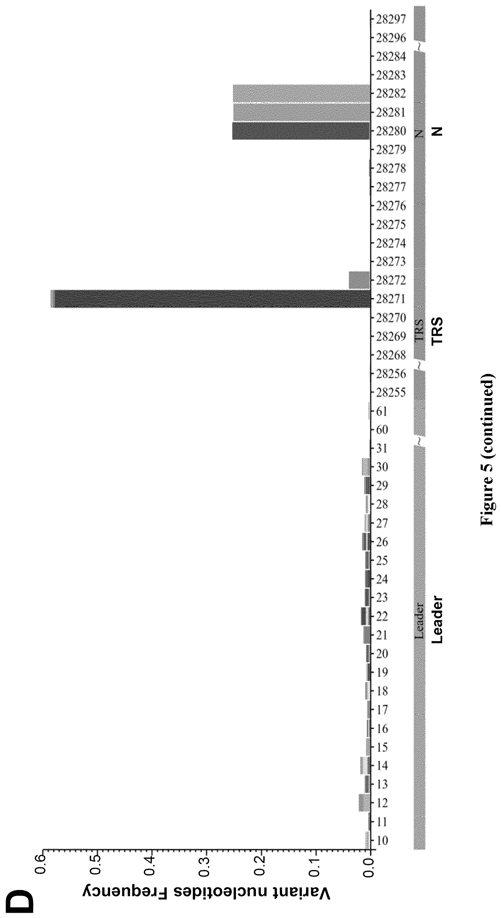

As sgN is the most abundant sgRNA during SARS-CoV-2 infection, and due to its pivotal role in viral genome packaging, we set-out here to transiently transfer into the host cells (i.e., HEK293T-ACE2) an oligonucleotide as anti-sense against the TRS sequence linked to the first ATG corresponding to the most 5' end of the of N gene (following the leader sequence). We then infected these cells with VOC alpha (B1.1.7) SARS-CoV-2 virus, to thus measure sgN, E, N, and sgE with the intent to quantify their virus load. For this purpose, we used sense and antisense 2'-O-methyl RNA oligonucleotides that targeted part of the leader sequence of SARS-CoV-2 followed by TRS and the 5' end of gene N (Figure 1A, within the arrow with dashed lines). This sequence was confirmed by Sanger sequence analysis from a swab sample from a COVID19-affected patient (Fig. 5A). We designed the sequence after aligning it with all of the SARS-CoV-2 variants identified to date. Figure 1B shows the sequence alignment with the nucleotides that comprise the designed 2'-O-methyl RNAs aligned with each independent VOC variant showing nucleotide variations. The sequence identities seen were 81.1% for Alpha, 100% for Beta, 100% for Gamma, 89.2% for Delta, and 97.3% for Omicron (see Figure 1B and Figure 5B-D). As additional help to draw the oligonucleotide sequence, we obtained sequencing data from an RNA Nanopore sequencing approach using Vero E6 monkey kidney cells previously infected with the 20A clade SARS-CoV-2 virus. These analyses allowed the determination of the best match sequence junction between the leader and TRS-B sequences of sgN (see Figure 1A, B). Of importance the sequence was also confirmed with 100% percentage identity in the sgN sequence with a frequency of 82.2% on a total 5,781 sgN sequences obtained by the Nanopore data (VeroE6 cells infected with SARS-CoV-2-20A at 0.1 MOI for 60 h of infection). These sequences have been deposited at Sequence Read Archive SRA - BioProject PRJNA688696, see additional data.

The synthesis and purification of the 2'-O-methyl sense or antisense RNA nucleotide was carried out following the procedures described in Material and Methods. We then transfected 2'-O-methyl sense or antisense RNA (1 mg) into HEK-293T cell clones stably overexpressing human ACE2 (i.e., HEK293T-ACE2 cells; Figure 1C). Twelve hours from start of transfection, the cells were infected with B.1.1.7 VOC Alpha SARS-CoV-2 viral particles (0.03 MOI) for a further 36 h (Figure 1C). Of importance, these virus particles contained the virus RNA sequence (sgN) that shows 81.1% percentage identity to the sequence of the 2'-O-methyl antisense RNA. Immunoblotting analysis showed a statistically significant decrease in viral N protein levels in these SARS-CoV-2-infected cells previously transfected with 2'-O-methyl antisense RNA (Figure ID). To ascribe the reduced N protein levels to the impairment of sgN transcripts, we also performed qPCR analysis using the SYBR green and Taqman methodologies (see Material and Methods) to detect the viral gene transcripts. The data presented in Figure IE show specific reduction of sgN levels by the 2'-O-methyl antisense RNA. This thus confirmed that although there was 81.1% percentage identity between the 2'-O-methyl antisense RNA and the sgN antisense technology, this percentage identity was sufficient to impair sgN translation (Figure IE). As a consequence, the levels of other SARS-CoV-2 genes (i.e., gene s E, N1-3) were also decreased (Figure IE) only in the infected cells that expressed 2'-O-methyl antisense RNA. At this time, this phenomenon can be ascribed to: (i) a reduction in the N protein, which has a role in the discontinuous transcription mechanism; and (ii) a substantial reduction in virus replication after 36 h of infection.

### Example 5 - Reduction of syncytia formation by targeting sgN in HEK293T-ACE2 SARS-CoV-2-infected cells

Syncytia phenomena are related to the persistence viral RNA infection and replication in SARS-CoV-2-infected patients. Syncytia can also be induced by certain types of infections by viruses, such as human immunodeficiency virus, respiratory syncytial virus, and herpes simplex virus. SARS-CoV-2 virus-induced cell fusion facilitates the transfer of viral genomes to neighboring cells. In lymphocytes in the human lung, widespread syncytia formation has been seen between SARS-COV-2-infected pneumocytes and healthy pneumocytes in patients infected with SARS-CoV-2. A model through ACE2 and S protein interaction was described showing how syncytia can be used to facilitate virus transmission. However, the viral and cellular mechanisms that regulate the formation of syncytia during SARS-CoV-2 infection remains largely elusive. We asked here whether syncytia formation was inhibited by transfection with 2'-O-methyl antisense RNA, as the cellular model of infection (HEK293T-ACE2 cells), mainly because the S protein is very low in cells infected by SARS-CoV-2, and this would affect syncytia formation by impairing the ACE2-S protein interactions that prime syncytia formation. Indeed, upon SARS-CoV-2 infection, the cells that received 2'-O-methyl antisense RNA showed less S protein expression, and as a consequence, less syncytia formation, as revealed by high-resolution confocal microscopy (Figure 2A). The additional measure of proportion of syncytia further demonstrated this impaired of syncytia formation (P <0.001 versus the control; P <0.01 versus the sense 2'-O-methyl RNA treated cells; see Figure 2B). Altogether, these data show that the biogenesis of sgN goes through a "leader to TRS body fusion model" that produces sufficient copies of new sgN mRNAs to translate then into N proteins that are used by the gRNA to be package into nascent virions. We show here that in vitro it is possible to impair this process using 2'-O-methyl antisense RNA oligos, with therapeutic benefits seen through reduction of viral load in SARS-CoV-2-infected cells, thus further inhibiting syncytia formation and subsequent virus propagation (Figure 3A). Then the model describes that during SARS-CoV-2 infections, the acute phase of virus replication is enhanced by expression of viral genome and subgenomic transcripts (Figure 3B); while during the negativization process, the loss of sgN detection is seen (i.e., undetectable levels: Cq >40; viral ORFlab and E Cq values >33.15 and 33.16, respectively).

### Example 6 - experimental procedures

### Cell culture

HEK-293T cells (CRL-3216, ATCC), HEK-293T stable clones overexpressing human ACE2 (HEK293T-ACE2) and Vero E6 cells (C1008, ATCC) were grown in a humidified 37°C incubator with 5% CO₂. The cells were cultured in feeder-free conditions using Dulbecco's modified Eagle's medium (41966-029; Gibco) with 10% fetal bovine serum (10270-106; Gibco), 2 mM L-glutamine (25030-024; Gibco), and 1% penicillin/streptomycin (P0781; Sigma-Aldrich), with medium changed daily. Cells were dissociated with Trypsin-EDTA solution (T4049, Sigma-Aldrich) when the culture reached ~80% confluency.

### Generation of HEK293T-ACE2 stable clones

HEK-293T cells were plated in 6-well plates in 2 ml of Dulbecco's modified Eagle's medium (41966-029; Gibco) with 10% fetal bovine serum (10270-106; Gibco). When the culture reached ~70% confluency, they were transfected with pCEP4-myc-ACE2 plasmid (#141185, Addgene) with X-tremeGENE 9 DNA Transfection Reagent (06365779001; Sigma-Aldrich). Briefly, X-tremeGENE 9 DNA Transfection Reagent was equilibrated at room temperature (+15 to +25°C) and diluted with serum-free Dulbecco's modified Eagle's medium (41966-029; Gibco) to a concentration of 3 µl reagent/100 µl medium. Then, 1 µg of DNA plasmid was added to 100 µl of diluted X-tremeGENE 9 DNA Transfection Reagent (3:1 ratio [µl]). The transfection reagent:DNA complex was then incubated for 15 minutes at room temperature. Finally, the transfection complex was added to the cells in a dropwise manner. Following forty-eight hours from transfection, the cell culture medium was changed, and the cells clones were selected using 800 mg/ml hygromycin.

### Transient transfection with 2'-O-Methyl RNA oligos targeting sgN

### 2'-O-Methyl RNA oligos design

The following 2'-O-methyl RNA oligonucleotides were designed against the junction site between the leader sequence, the transcription-regulating sequences (TRSs) and the 5' end of gene N (leader-"junction"-TRS-"junction"-N) of SARS-CoV-2:
Sense: TCTCTAAACGAACAAACTAAAATGTCTGATAATGGAC (SEQ ID NO:10)
Antisense: GTCCATTATCAGACATTTTAGTTTGTTCGTTTAGAGA (SEQ ID NO:9)

To assess the initiation on the different viral transcripts, and enable leader-junction site unique alignment, we used RNA Nanopore sequencing data obtained from previously SARS-CoV-2 infected (20A clade) Vero E6 monkey kidney cells [5]. This sequence was further aligned with all of the SARS-CoV-2 variants identified to date, and was confirmed by Sanger sequence analysis from a swab sample from a COVID19-affected patient. These analyses allowed the determination of the best match sequence junction between the leader, TRS-B and the subgenomic N transcript (sgN). The 2'-O-methyl RNA oligonucleotides were synthetized at DNA lab Facility at CEINGE, Biotecnologie Avanzate (Naples).

### 2'-O-Methyl RNA oligos targeting sgNtransfection in HEK293T-ACE2 cells before infection with SARS-CoV-2

HEK293T-ACE2 cells were transfected with 1 mg of sense or anti-sense 2'-O-methyl RNA oligonucleotides against SgN. Transient transfections were performed with X-tremeGENE 9 DNA Transfection Reagent (06365779001; Sigma-Aldrich). To this end, X-tremeGENE 9 DNA Transfection Reagent was equilibrated at room temperature and diluted with serum-free Dulbecco's modified Eagle's medium (41966-029; Gibco) (3 µl reagent/100 µl medium). Then, 1 µg of sense or anti-sense 2'-O-methyl RNA oligonucleotides were added to 100 µl of diluted X-tremeGENE 9 DNA Transfection Reagent (3:1 ratio [µl]). The transfection reagent:RNA complex was incubated for 15 minutes at room temperature. The transfection complex was then added to the cells in a dropwise manner. Twelve hours after transfection, the cell culture medium was changed, and the cells were infected with SARS-CoV-2 particles (GISAID accession number: EPI_ISL_736997). Forty-eight hours after transfection (i.e., after 36 h of infection), the HEK293T-ACE2 cells were lysed.

### SARS-CoV-2 isolation and infection

SARS-CoV-2 was isolated from a nasopharyngeal swab obtained from an Italian patient sample as previously described [5]. Briefly, Vero E6 cells (8 × 105) were trypsinized and resuspended in Dulbecco's modified Eagle's medium (41966-029; Gibco) with 2% FBS in T25 flask to which the clinical specimen (100 ml) was added. The inoculated cultures were grown in a humidified 37°C incubator with 5% CO₂. Seven days after infection, when cytopathic effects were observed, the cell monolayers were scrapped with the back of a pipette tip, while the cell culture supernatant containing the viral particles was aliquoted and frozen at -80°C. Viral lysates were used for total nucleic acid extraction for confirmatory testing and sequencing (GISAID accession number: EPI_ISL_736997).

HEK-293T cell clones stably overexpressing ACE2 (8 ×10⁵ cells) were plated in T25 flasks for transfection with sense or antisense 2'-O-methyl RNA oligos targeting sgN. After 12 h, the cell culture medium was changed, and the 2'-O-methyl RNA transfected cells were then infected with viral particles of the 20I/501Y.V1 (B.1.1.7) clades (0.03 MOI), (GISAID accession number: EPI_ISL_736997). Uninfected cells were used as the negative control. After 36 h of infection, the cells were lysed or fixed. These experiments were performed in a BLS3-authorized laboratory.

### RNA extraction and qPCR from HEK-293T cells overexpressing ACE2

RNA samples were extracted with TRIzol RNA Isolation Reagent according to the manufacturer instructions. Reverse transcription was performed with 5× All-In-One RT MasterMix (catalog no. g486; ABM), according to the manufacturer instructions. The reverse transcription products (cDNA) were amplified by qRT-PCR using an RT-PCR system (7900; Applied Biosystems, Foster City, CA, USA). The cDNA preparation was through the cycling method by incubating the complete reaction mix as follows:
∘ cDNA reactions: [25 °C for 5 min and 42 °C for 30 min]
∘ Heat-inactivation: 85°C for 5 min
∘ Hold stage: 4 °C

*Viral N and human RNAse P detection.* The detection of viral N gene and human RNase P was performed by using the in-vitro diagnostics IVD-approved approved "quanty COVID19" kit (RT-25; Clonit). This diagnostic kit provides a specific quantitative detection of the viral N1, N2, and N3 fragments (from the SARS-CoV-2 N gene) and human RNaseP gene by using differentially labeled target probes into two mixes (First mix: N1, FAM; N2, VIC; Second mix: N3, VIC; RNase P, CY5). These runs were performed on a PCR machine (CFX96; Bio-Rad; IVD approved) according to manufacturer instructions. Briefly, 5 ml RNA was used for the following thermal protocol below:
∘ UNG incubation: 25°C for 2 min
∘ Reverse Transcription: 50°C for 15 min
∘ Inactivation/ Denaturation: 95 °C for 2 min
∘ Denaturation and Annealing (×45 cycles): [95°C for 3 sec and 55 °C for 30 sec].

The quantification cycle (Cq) values of N1, N2, and N3 are reported as means ±SD normalized to the internal control (human RNase P) of three replicates.

*Viral E, human ACE2 and b-Actin (ACTB) detection (SYBR green).* The targets E, ACE2 and ACTB were detected with SYBR green approach by using BrightGreen 2X qRT-PCR MasterMix Low-ROX (MasterMix-LR; ABM). Human ACTB was used as the housekeeping gene used to normalize the quantification cycle (Cq) values of the other genes. These runs were performed on a PCR machine (Quantstudio5, Lifetechnologies) with the following thermal protocol:
∘ Hold stage: 50°C for 2 min
∘ Denaturation Step: 95 °C for 10 min
∘ Denaturation and Annealing (×45 cycles): [95°C for 15 sec and 60 °C for 60 sec].
∘ Melt curve stage: [95°C for 15 sec, 60°C for 1 min and 95°C for 15 sec]

The details of the primers used in these SYBR green assays are provided below:
E Forward (SYBR green): ACAGGTACGTTAATAGTTAATAGCGT (SEQ ID NO:11)
E Reverse (SYBR green): ATATTGCAGCAGTACGCACACA (SEQ ID NO:12)
ACE2 Forward (SYBR green): GAAATTCCCAAAGACCAGTGGA (SEQ ID NO:13)
ACE2 Reverse (SYBR green): CCCCAACTATCTCTCGCTTCAT (SEQ ID NO:14)
ACTB Forward (SYBR green): GACCCAGATCATGTTTGAGACCTT (SEQ ID NO:15)
ACTB Reverse (SYBR green): CCAGAGGCGTACAGGGATAGC (SEQ ID NO:16)

The relative expression of the target genes was determined using the 2-ΔΔCq method, as the fold increase compared with the controls. The data are presented as means ±SD of the 2-ΔΔCq values (normalized to human ACTB) of three replicates.

*Viral sgN, sgE and human b-Actin (ACTB) detection (Taqman).* The target sgN, sgE and ACTB were detected with Taqman approach [5]. These runs were performed on a PCR machine (Quantstudio 12K Flex, Appliedbiosystems) with the following thermal protocol:
∘ Denaturation Step: 95 °C for 20 sec
∘ Denaturation and Annealing (×50 cycles): [95°C for 1 s and 60 °C for 20 s].

The details of the primers used in these Taqman assays are provided below:
sgN Forward (Taqman): CAACCAACTTTCGATCTCTTGTA (SEQ ID NO: 17)
sgN Reverse (Taqman): TCTGCTCCCTTCTGCGTAGA (SEQ ID NO: 18)
sgN Probe (Taqman): 5'FAM-ACTTCCTCAAGGAACAACATTGCCA-BBQ-3' (SEQ ID NO:19)
sgE Forward (Taqman): CGATCTCTTGTAGATCTGTTCTC (SEQ ID NO:20)
sgE Reverse (Taqman): ATATTGCAGCAGTACGCACACA (SEQ ID NO:21)
sgE probe (Taqman): 5'FAM-ACACTAGCCATCCTTACTGCGCTTCG-BBQ-3' (SEQ ID NO:22)
ACTB Forward (Taqman): Hs01060665_g1 (Thermo Fisher Scientific)

The quantification cycle (Cq) values of sgN and sgE are reported as means ±SD normalized to the control (human ACTB) of three replicates.

### Sanger sequencing

The cDNA was obtained by random primer RT-PCR using SensiFASTcDNA synthesis kits (Bioline, provided by Life Technologies Italia, Monza MB, Italy), using 5 µL RNA extracted from nasopharyngeal swabs. We used the primer setting (sgN-For CAACCAACTTTCGATCTCTTGTA (SEQ ID NO:23) and sgN-Rev TCTGGTTACTGCCAGTTGAATC (SEQ ID NO:24)) to amplify the sgN region, and to perform the Sanger sequencing.

### Immunoblotting

Cells were lysed in 20 mM sodium phosphate, pH 7.4, 150 mM NaCl, 10% (v/v) glycerol, 1% (w/v) sodium deoxycholate, 1% (v/v) Triton X-100, supplemented with protease inhibitors (Roche). The cell lysates were cleared by centrifugation at 16,200× g for 30 min at room temperature, and the supernatants were removed and assayed for protein concentrations with Protein Assay Dye Reagent (BioRad). The cell lysates (20 µg) were resolved on 10% SDS-PAGE gels. The proteins were transferred to PVDF membranes (Millipore). After 1 h in blocking solution with 5% (w/v) dry milk fat in Tris-buffered saline containing 0.02% [v/v] Tween-20, the PVDF membranes were incubated with the primary antibody overnight at 4 °C: anti-ACE2 (1:1000; ab15348), anti-SARS-CoV-2 N protein (1:250; 35-579; ProSci Inc.), or anti-β-actin (1:10000; A5441; Sigma). The membranes were then incubated with the required secondary antibodies for 1 h at room temperature: secondary mouse or rabbit horseradish-peroxidase-conjugated antibodies (NC 15 27606; ImmunoReagents, Inc.), diluted in 5% (w/v) milk in TBS-Tween. The protein bands were visualized by chemiluminescence detection (Pierce-Thermo Fisher Scientific Inc., IL, USA). Densitometry analysis was performed with the ImageJ software. The peak areas of the bands were measured on the densitometry plots, and the relative proportions (%) were calculated. Then, the density areas of the peaks were normalized with those of the loading controls, and the ratios for the corresponding controls are presented as fold-changes. Immunoblotting was performed in triplicate. Densitometry analyses shown were derived from three independent experiments.

### Immunofluorescence

SARS-CoV-2-infected HEK293T-ACE2 cells were fixed in 4% paraformaldehyde in phosphate-buffered saline (PBS) for 30 min, washed three times with PBS, and permeabilized for 15 min with 0.1% Triton X-100 (215680010; Acros Organics) diluted in PBS. The cells were then blocked with 3% bovine serum albumin (A9418; Sigma) in PBS for 1 h at room temperature. The samples were incubated with the appropriate primary antibodies overnight at 4 °C: anti-ACE2 (1:1000; ab15348; Abcam), anti-SARS S protein (1:100; ab272420; Abcam). After washing with PBS, the samples were incubated with the secondary antibody at room temperature for 1 h: anti-mouse Alexa Fluor 488 (1:200; ab150113; Abcam) or anti-rabbit Alexa Fluor 647 (1:200; ab150075; Abcam). DNA was stained with DAPI (1:1000; #62254; Thermo Fisher). The slides were washed and mounted with cover slips with 50% glycerol (G5150; Sigma-Aldrich). Microscopy images were obtained using the Elyra 7 platform (Zeiss) with the optical Lattice SIM² technology (with the ZEN software, Zeiss, blue edition), using the 63× oil immersion objective.

### Statistical analysis

Statistical significance was defined as *P* <0.05 by unpaired two-tailed Student's t-tests. All of the data are given as means ±SD. In the Figures, statistical significance is represented as follows: ^{∗}*P* <0.05, ^{∗∗}*P* <0.01, and ^{∗∗∗}*P* <0.001.

### REFERENCES

- Li T, Zhang Y, Fu L, Yu C, Li X, Li Y, et al. siRNA targeting the leader sequence of SARS-CoV inhibits virus replication. Gene Ther. 2005;12(9):751-61. Epub 2005/03/18. doi: 10.1038/sj.gt.3302479. PubMed PMID: 15772689; PubMed Central PMCID: PMCPMC7091583].
- Lin SY, Liu CL, Chang YM, Zhao J, Perlman S, Hou MH. Structural basis for the identification of the N-terminal domain of coronavirus nucleocapsid protein as an antiviral target. J Med Chem. 2014;57(6):2247-57. Epub 2014/02/26. doi: 10.1021/jm500089r. PubMed PMID: 24564608; PubMed Central PMCID: PMCPMC3983370.
- De Antonellis et al, MicroRNA 199b-5p delivery through stable nucleic acid lipid particles (SNALPs) in tumorigenic cell lines. (2013) Naunyn-Schmiedeberg's Arch Pharmacol DOI 10.1007/s00210-013-0837-4.
- Zoulikha Makhloufi et al., Pulmonary delivery of siRNA against acute lung injury/acute respiratory distress syndrome, Acta Pharmaceutica Sinica B, https://doi.org/10.1016/j.apsb.2021.08.009.
- Kim D, Lee JY, Yang JS, Kim JW, Kim VN, Chang H. The Architecture of SARS-CoV-2 Transcriptome. Cell. 2020;181(4):914-21 870 e10. Epub 2020/04/25. doi: 10.1016/j.cell.2020.04.011. PubMed PMID: 32330414; PubMed Central PMCID: PMCPMC7179501.

## Claims

1. An antisense oligonucleotide having a sequence complementary to a region of SARS-CoV2 subgenomic RNA encoding nucleoprotein N (sgN), said region consisting of a leader sequence (LS), two transcription-regulatory sequences (TRS), namely TRS(leader) and TRS(body), and a 5' portion of gene N (N), such that:
5'-LS-TRS(leader)-TRS(body)-N-3'
wherein:
- LS is UCGAUCUCUUGUAGAUCUGUUCU (SEQ ID NO:1) or a fragment thereof including or consisting of UCU at the 3'end
- TRS(leader) is CUAAACGAAC (SEQ ID NO:2)
- TRS(body) is selected from AAACUAAA; CAAACUAA and AAACUUAA
- N is selected from AUGUCUGAUAAUGGAC (SEQ ID NO:3) and AUGUCUCUAAAUGGAC (SEQ ID NO:4).

2. An antisense oligonucleotide according to claim 1, wherein:
- LS is UCU
- TRS(leader) is CUAAACGAAC (SEQ ID NO:5)
- TRS(body) is AAACUAAA
- N is AUGUCUGAUAAUGGAC (SEQ ID NO:6).

3. An antisense oligonucleotide according to claims 1-2, which comprises from 30 to 40 bases.

4. An antisense oligonucleotide according to claims 1-3, consisting of the sequence GXCCAXXAXCAGACAXXXXAGXXXGXXCGXXXAGAGA (SEQ ID NO:7) wherein "X" bases are, independently from one another, U or T.

5. An antisense oligonucleotide according to claim 4, consisting of a sequence which is selected from:
GUCCAUUAUCAGACAUUUUAGUUUGUUCGUUUAGAGA (SEQ ID NO:8)
and
GTCCATTATCAGACATTTTAGTTTGTTCGTTTAGAGA (SEQ ID NO:9).

6. An antisense oligonucleotide according to claims 1-5, which comprises a backbone with modified nucleotides to prevent oligonucleotide enzymatic cleavage or degradation.

7. An antisense oligonucleotide according to claim 6, **characterized in that** said backbone includes a modified nucleotide which is selected from phosphorodiamidate morpholino (PMO) nucleotide, 5' -phosphorothioate nucleotide, 5-methylated cytosine nucleotide, 2'-O-methyl nucleotide, 2'-O-methoxyethyl nucleotide, 2'-fluoro nucleotide, 3'-nitrogen nucleotide; or said backbone includes a locked nucleic acid (LNA) or a peptide nucleic acid (PNA).

8. A pharmaceutical composition comprising an antisense oligonucleotide according to claims 1-7.

9. A composition according to claim 8, which is in a form suitable for delivery of the oligonucleotide to the respiratory tract and to the lungs.

10. A composition according to claim 9, wherein said form is suitable for administration via nasal or pulmonary routes.

11. An oligonucleotide according to claims 1-7 or a composition according to claims 8-10, for use in the prevention or treatment of a coronavirus infection.

12. An oligonucleotide or a composition for use according to claim 11, wherein said coronavirus is SARS-CoV-2.
